# EUROPEAN PATENT APPLICATION

(11) **EP 4 177 243 A1**
(43) Date of publication of application: **10.05.2023**
(21) Application number: 21828493.3
(22) Date of filing: 27.05.2021
(51) Int. Cl.: C07D 215/28, C07D 401/12, A61K 31/47, A61P 35/00

(54) **PREPARATION METHOD FOR AROMATIC ETHER COMPOUND**

(30) Priority: 22.06.2020 CN 202010573569
(71) Applicant: Jiangsu Yahong Meditech Co., Ltd., Taizhou, Jiangsu 225316 (CN); Asieris Pharmaceuticals (Shanghai) Co., Ltd., Pudong, Shanghai 201203 (CN)
(72) Inventor: WU, Liang, Taizhou, Jiangsu 225316 (CN); ZHOU, Chen, Taizhou, Jiangsu 225316 (CN); DENG, Yijun, Taizhou, Jiangsu 225316 (CN)
(74) Representative: EP&C
(86) International application number: PCT/CN2021/096279
(87) International publication number: WO 2021/258979

(57) **Abstract**

A preparation method for an aromatic ether compound. The preparation method comprises the following step: c) in a solvent, in the presence of a catalyst and alkali, a compound V reacts with a compound 6 to obtain a compound VII, 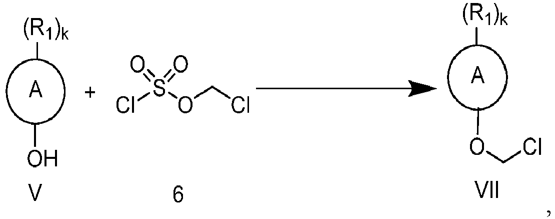 wherein the solvent is a solvent system having two or more elements selected from water, tetrahydrofuran, 2-methyltetrahydrofuran, ethyl acetate, and dioxane. According to the preparation method for the aromatic ether compound in the present invention, the conversion rate of the reaction can be greatly improved, and thus the yield of the step and the total yield of the whole reaction route are improved; post-treatment and purification operations for preparing the compound VII are greatly simplified, and the compound VII having high purity can be obtained only be simply filtering a reaction solution and washing and drying a filter cake. The preparation method is suitable for industrial scale-up production.

## Description

### FIELD OF THE INVENTION

The present invention relates to a preparation method for an aromatic ether compound.

### BACKGROUND OF THE INVENTION

Nitroxoline, a commercially available antibacterial drug, has long been used in the treatment of urinary tract infections. Recent discoveries have shown that nitroxoline is also very effective in inhibiting angiogenesis and inhibiting the growth and invasion of cancer cells, and is currently being developed for anti-tumor applications. Human pharmacokinetic studies have shown that nitroxoline can be rapidly absorbed into the blood circulation. However, due to the severe first-pass effect of the liver on the drug, its biological half-life is very short (a single-arm, open-label, multi-center phase II clinical trial conducted by Jiangsu Yahong Meditech Technology Co., Ltd. in China has shown that its half-life is 1.22-1.44 hours), thus, frequent administration is required. In order to maintain continuous drug exposure, nitroxoline drugs are generally prescribed to be administered three times a day (TID) or four times a day (QID), which not only brings economic losses, is not conducive to patient compliance, but increases the persistent damage of the drug to the body as a more severe consequence. Meanwhile, due to the very low water solubility of nitroxoline, it is often necessary to prepare it as an immediate-release formulation to improve the solubility, which virtually increases the production cost.

A prodrug is a compound obtained by chemical modification of an active drug, which is converted into the original drug *in vivo* by the action of enzymes to exert its efficacy. Prodrugs are widely used in drug research and development, and they have been successfully developed for many different drugs with good effects in application. The prodrug strategy can solve some defects of the active agent due to its own physical and chemical properties, for example: 1) eliminating the bad odor of the drug; 2) increasing the blood concentration of the drug; 3) improving the lipid solubility or water solubility of the drug; 4) prolonging the action time of the drug; 5) changing the administration route of the drug, and the like.

((5-Nitroquinolin-8-yl)oxy)methylisobutyryl-L-prolinate is a prodrug of nitroxoline, which can solve the above-mentioned defects of nitroxoline. Currently, only patent application WO 2020/063824 A1 discloses a preparation method for ((5-nitroquinolin-8-yl)oxy)methylisobutyryl-L-prolinate, and the preparation method is detailed as follows:

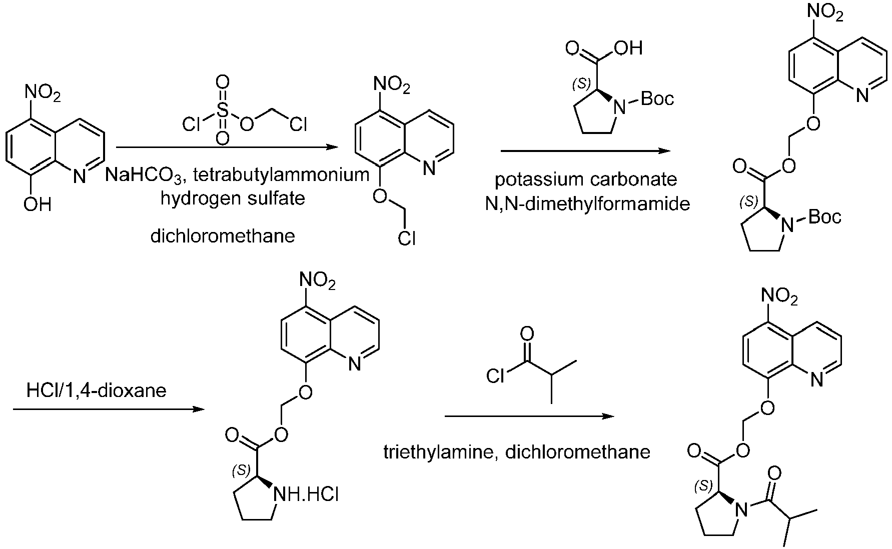

However, in the above-mentioned preparation method, the conversion rate of the first reaction step is low, which in turn leads to a low yield of this step, and ultimately leads to a low yield of the overall synthesis route.

### SUMMARY OF THE INVENTION

In order to solve the technical problem of low conversion rate of the first reaction step in the above-mentioned preparation method, the present invention provides a preparation method for an aromatic ether compound.

Thus, an object of the present invention is to provide a preparation method for the compound of formula VII, comprising the following step of:
c) reacting compound V with compound 6 in a solvent in the presence of a catalyst and a base to obtain the compound VII, 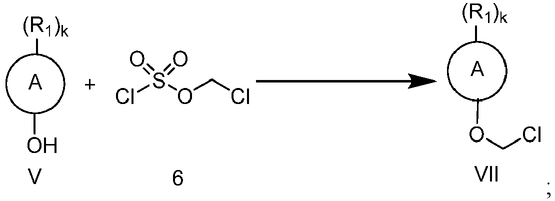
wherein, ring A is an aromatic ring or a heteroaromatic ring, preferably a 6- to 10-membered aromatic ring or a 5- to 10-membered heteroaromatic ring, more preferably a benzene ring, a naphthalene ring, a pyridine ring or a quinoline ring; the hydroxyl group is attached to a carbon on ring A;
each R₁ is independently selected from the group consisting of -R₂, 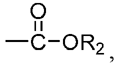 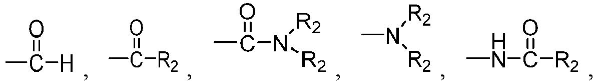 -NO₂, -NO, 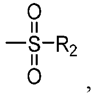 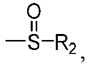 -S-R₂, -OR₂ and -X; wherein, each R₂ is independently C₁-C₂₀ alkyl, preferably C₁-C₆ alkyl, more preferably methyl, ethyl, n-propyl or isopropyl; X is halogen, preferably fluorine, chlorine, bromine or iodine;
k is from 0 to the maximum number available for substitution on ring A; preferably, k is an integer from 0 to 6; more preferably, k is an integer from 0 to 4; further preferably, k is 0, 1 or 2; most preferably, k is 1;
the solvent is a binary or more solvent system in which the solvent used is selected from the group consisting of water, tetrahydrofuran, 2-methyltetrahydrofuran, ethyl acetate and dioxane.

In a preferred embodiment of the present invention, the preparation method for the compound of formula VII according to the present invention is provided, wherein, in step c), the compound V is 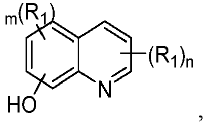 m and n are each independently 0, 1 , 2 or 3; preferably 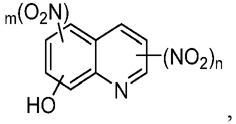 m and n an e each independently 0, 1, 2 or 3; more preferably 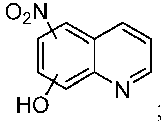 further more preferably

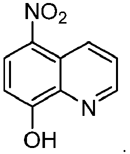

In another preferred embodiment of the present invention, the preparation method for the compound of formula VII according to the present invention is provided, wherein, in step c), the compound V is 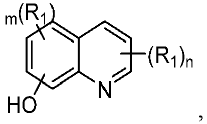 the compound VII is 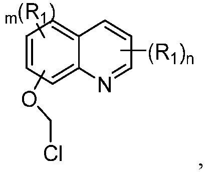 m and n are each independently 0, 1, 2, or 3;
preferably, the compound V is 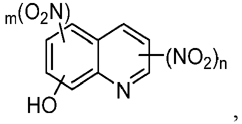 the compound VII is 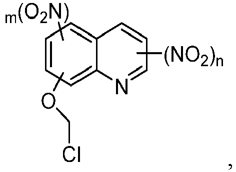 m and n are each independently 0, 1, 2 or 3;
more preferably, the compound V is 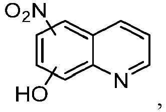 the compound VII is 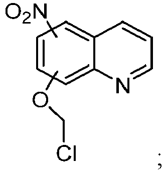
further more preferably, the compound V is 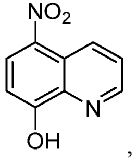 the compound VII is

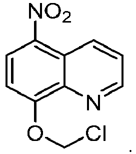

In another preferred embodiment of the present invention, the preparation method for the compound of formula VII according to the present invention is provided, wherein, in step c), the halogen is fluorine, chlorine, bromine or iodine.

In another preferred embodiment of the present invention of the preparation method for the compound of formula VII according to the present invention is provided, wherein, in step c), the solvent is a binary solvent system or ternary solvent system in which the solvent used is selected from the group consisting of water, tetrahydrofuran, 2-methyltetrahydrofuran, ethyl acetate and dioxane.

In another preferred embodiment of the present invention, the preparation method for the compound of formula VII according to the present invention is provided, wherein, in step c), the solvent is any one of the binary solvent systems of water/tetrahydrofuran, water/2-methyltetrahydrofuran and water/ethyl acetate; or, any one of the ternary solvent systems of water/2-methyltetrahydrofuran/tetrahydrofuran and water/2-methyltetrahydrofuran/dioxane; the solvent is preferably a binary solvent system of water/tetrahydrofuran or a ternary solvent system of water/2-methyltetrahydrofuran/tetrahydrofuran; more preferably a ternary solvent system of water/2-methyltetrahydrofuran/tetrahydrofuran. In the binary solvent system, the volume ratio of water to the organic solvent is preferably 10:15 to 15:0.1, more preferably 0.8:1 to 1.2:1, further more preferably 1:1. In the ternary solvent system, the volume ratio of the three is preferably 10:15:15 to 15:5:5, more preferably 3:2:1 to 2.25:0.5:1, further more preferably 2:1:1; wherein, "the volume ratio of the three" refers to the volume ratio of the three substances appearing in sequential order in the above-mentioned ternary solvent system.

In another preferred embodiment of the present invention, the preparation method for the compound of formula VII according to the present invention is provided, wherein, in step c), the catalyst is a conventional quaternary ammonium phase transfer catalyst, preferably one or more of tetrabutylammonium hydroxide, tetrabutylammonium acetate, tetrabutylammonium hydrogen sulfate and tetrabutylammonium chloride, more preferably tetrabutylammonium hydroxide.

In another preferred embodiment of the present invention, the preparation method for the compound of formula VII according to the present invention is provided, wherein, in step c), the base is one or both of sodium bicarbonate and potassium bicarbonate, preferably sodium bicarbonate.

In another preferred embodiment of the present invention, the preparation method for the compound of formula VII according to the present invention is provided, wherein, in step c), the molar ratio of the compound V to the catalyst is 1:0.01 to 1:0.3, preferably 1:0.05 to 1:0.2, more preferably 1:0.1.

In another preferred embodiment of the present invention, the preparation method for the compound of formula VII according to the present invention is provided, wherein, in step c), the molar ratio of the compound V to the base is 1:2.5 to 1:15, for example 1:3, preferably 1:6 to 1:10, more preferably 1:6 to 1:8, further more preferably 1:7 to 1:8.

In another preferred embodiment of the present invention, the preparation method for the compound of formula VII according to the present invention is provided, wherein, in step c), the molar ratio of the compound V to the compound 6 is 1:1 to 1:5, preferably 1:2 to 1:3, more preferably 1:2.5 to 1:2.7.

In another preferred embodiment of the present invention, the preparation method for the compound of formula VII according to the present invention is provided, wherein, in step c), the volume/mass ratio of the solvent to the compound V is 20 mL/g to 50 mL/g, preferably 24 mL/g to 45 mL/g, more preferably 25 mL/g to 40 mL/g, further more preferably 25 mL/g to 35 mL/g, most preferably 30 mL/g.

In another preferred embodiment of the present invention, the preparation method for the compound of formula VII according to the present invention is provided, wherein, in step c), the reaction temperature of the reaction is 20°C to 35°C, preferably 25°C to 30°C.

In another preferred embodiment of the present invention, the preparation method for the compound of formula VII according to the present invention is provided, wherein, the preparation method can further comprise the following step before step c):
b) hydrolyzing compound 3 in a solvent in the presence of a base to obtain compound 4, 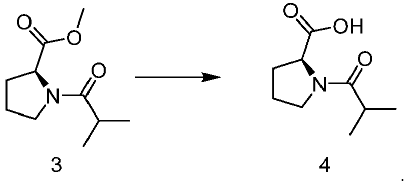 in step b), the solvent is preferably a mixed solvent of 2-methyltetrahydrofuran and water; the base is preferably lithium hydroxide.

The preparation method can further comprise the following step before step b):
a) reacting compound 1 with compound 2 in a solvent in the presence of a base to obtain compound 3,

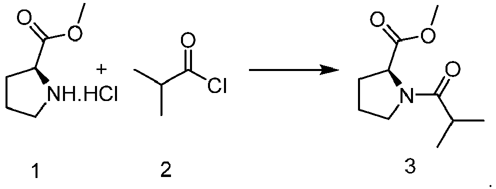

In step a), the solvent is preferably dichloromethane; the base is preferably one or both of triethylamine and diisopropylethylamine, more preferably triethylamine.

The present invention further provides a preparation method for a compound of formula VIII, characterized in that, the preparation method comprises the following step of:
d) reacting compound 4 with compound VII in a solvent in the presence of a base to obtain compound VIII, 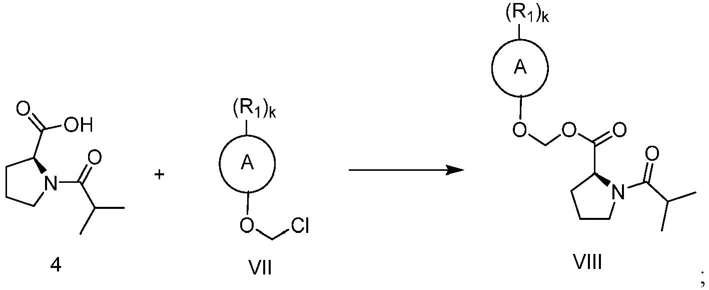
wherein, ring A is an aromatic ring or a heteroaromatic ring, preferably a 6- to 10-membered aromatic ring or a 5- to 10-membered heteroaromatic ring, more preferably a benzene ring, a naphthalene ring, a pyridine ring or a quinoline ring; -OCH₂Cl is attached to a carbon on ring A;
each R₁ is independently selected from the group consisting of -R₂, 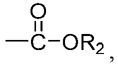 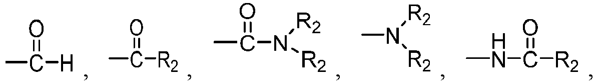 -NO₂, -NO, 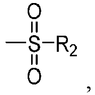 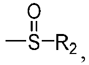 -S-R₂, -OR₂ and -X; wherein, each R₂ is independently C₁-C₂₀ alkyl, preferably C₁-C₆ alkyl, more preferably methyl, ethyl, n-propyl or isopropyl; X is halogen, preferably fluorine, chlorine, bromine or iodine;
k is from 0 to the maximum number available for substitution on ring A; preferably, k is an integer from 0 to 6; more preferably, k is an integer from 0 to 4; further preferably, k is 0, 1 or 2; most preferably, k is 1;
the compound VII is prepared according to the preparation method for the compound of formula VII according to the present invention.

In a preferred embodiment of the present invention, the preparation method for the compound of formula VIII according to the present invention is provided, wherein, in step d), the solvent is one or more of DMF, NMP and ACN, preferably DMF.

In another preferred embodiment of the present invention, the preparation method for the compound of formula VIII according to the present invention is provided, wherein, in step d), the base is one or more of potassium carbonate, cesium carbonate and sodium carbonate, preferably potassium carbonate.

In another preferred embodiment of the present invention, the preparation method for the compound of formula VIII according to the present invention is provided, wherein, in step d), the molar ratio of the compound 4 to the base is 1-1.5:1, preferably 1.2:1.

In another preferred embodiment of the present invention, the preparation method for the compound of formula VIII according to the present invention is provided, wherein, in step d), the molar ratio of the compound 4 to the compound VII is preferably 1-1.5:1, more preferably 1.2:1.

In another preferred embodiment of the present invention, the preparation method for the compound of formula VIII according to the present invention is provided, wherein, in step d), the ratio of the volume of the solvent to the mass of the compound 4 is 8:1 mL/g to 12:1 mL/g, preferably 10: 1 mL/g.

In another preferred embodiment of the present invention, the preparation method for the compound of formula VIII according to the present invention is provided, wherein, in step d), the reaction temperature of the reaction is 20°C to 30°C.

The preparation method for the compound of formula VIII according to the present invention is provided, wherein it also comprises a step e) of separating and purifying compound 8 after step d). The step e) preferably comprises the following steps of: dissolving the compound VIII obtained in step d) in a positive solvent, and then mixing the solution with an anti-solvent to obtain a crystal form of compound VIII. The step e) more preferably comprises the following steps of: mixing the mixed solution obtained after the completion of the reaction described in step d) with water, and performing liquid-liquid separation to obtain an organic phase; extracting the organic phase with ethyl acetate, removing water, and then concentrating the organic phase under reduced pressure to obtain a crude product; dissolving the crude product in a positive solvent, and then mixing the solution with an anti-solvent to obtain a crystal form of compound VIII;
wherein, saturated brine can be used for removing water; preferably, after removing water with saturated brine, drying over anhydrous sodium sulfate is performed, and after filtration, the concentration under reduced pressure is performed;
wherein, the positive solvent is preferably ethyl acetate;
wherein, the anti-solvent is preferably petroleum ether.

In another preferred embodiment of the present invention, the preparation method for the compound of formula VIII according to the present invention is provided, wherein, the compound VII is 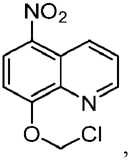 the compound VIII is

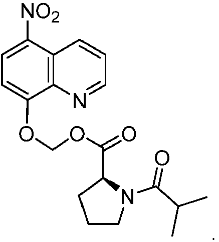

On the basis of not violating common knowledge in the art, the above-mentioned preferred conditions can be combined arbitrarily to obtain preferred examples of the present invention.

The reagents and materials used in the present invention are all commercially available.

The beneficial effect of the present invention is that the preparation method for the aromatic ether compound of the present invention can greatly improve the conversion rate of the above-mentioned first reaction step, thereby improving the yield of this step and the overall yield of the entire reaction route (the reaction route for producing compound VIII). The preparation method of the aromatic ether compound of the present invention greatly simplifies the post-treatment and purification operations for preparing the compound VII. The compound VII with high purity can be obtained by simply filtering the reaction solution and washing and drying the filter cake, and the compound VII in the filtrate can also be extracted by simply concentration and crystallization, thereby avoiding the industrially difficult purification means such as column chromatography. Therefore, the preparation method is suitable for industrial scale-up production.

### DETAILED DESCRIPTION OF THE INVENTION

The present invention will be described in more detail below with reference to the examples. The examples of the present invention are only used to illustrate the technical solutions of the present invention, but do not limit the essence and scope of the present invention.

Hereinafter, TEA is triethylamine; DCM is dichloromethane; 2-Me-THF is 2-methyltetrahydrofuran; TBAOH is tetra-n-butylammonium hydroxide; THF is tetrahydrofuran; DMF is N,N-dimethylformamide.

Hereinafter, the LCMS applies an Agilent 1260 infinity II liquid phase + G6125B single quadrupole mass spectrometer.

Hereinafter, the method for analyzing the purity of samples is as follows: gradient elution is performed by using a Kinetex EVO C18 (50×4.6 mm, 5 µm, 100Å) chromatographic column with acetonitrile-water as the mobile phase at a flow rate of 1.5mL/min and at detection wavelengths of 210 nm and 254 nm.

Hereinafter, the model of the hydrogen spectrometer is WNMR-I-400MHz.

Hereinafter, silica gel column chromatography was performed with Yantai Huanghai silica gel 200-300 mesh silica gel as carrier.

The synthetic route of the Examples is as follows:

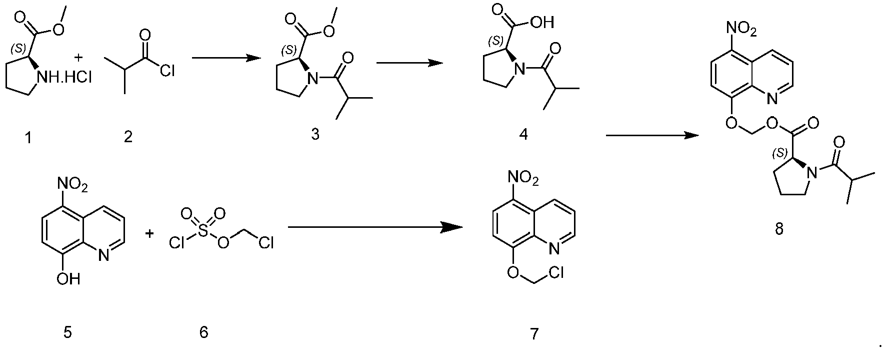

**Table 1 Experimental parameters and experimental results of the Examples and Comparative Examples**

| Example | Mass of Compound 5, eq. | Compound 6, eq. | Catalyst, eq. | Base, eq. | Solvent | Conversion rate/% | Yield /% | Purity /% |
|---|---|---|---|---|---|---|---|---|
| 1 | 1 g,1 eq | 2 eq | Tetrabutylammonium hydroxide, 0.1 eq | NaHCO₃, 6 eq | H₂O/THF, 20 mL/20 mL | 82.7 | / | |
| 2 | 1 g,1 eq | 2 eq | | NaHCO₃, 6 eq | H₂O/2-Me-THF, 20 mL/20 mL | 75.2 | / | |
| 3 | 1 g,1 eq | 2 eq | | NaHCO₃, 6 eq | H₂O/EA, 20mL/20mL | 73.8 | / | |
| 4 | 5 g,1 eq | 2.5 eq | | NaHCO₃, 7.5 eq | H₂O/THF, 100mL/100mL | 98.3 | / | |
| 5 | 5 g,1 eq | 2.5 eq | | KHCO₃,7.5 eq | H₂O/2-Me-THF, 100mL/100mL | 80 | / | |
| 6 | 10 g,1 eq | 2.7 eq | | NaHCO₃, 8.1 eq | H₂O/THF, 200mL/200mL | 77.9 | / | |
| 7 | 10 g,1 eq | 2.5 eq | | NaHCO₃, 7.5 eq | H₂O/THF, 200mL/200mL | 95.8 | / | |
| 8 | 10 g,1 eq | 2.5 eq | | NaHCO₃, 7.5 eq | H₂O/2-Me-THF/THF, 200mL/100mL/100mL | 98.7 | / | |
| 9 | 10 g,1 eq | 2.5 eq | | NaHCO₃, 7.5 eq | H₂O/2-Me-THF/THF, 150mL/75mL/75mL | 97.1 | / | |
| 10 | 10 g,1 eq | 2.5 eq | | NaHCO₃, 7.5 eq | H₂O/2-Me-THF/THF, 100mL/50mL/50mL | 85.8 | / | |
| 11 | 30 g,1 eq | 2.5 eq | | NaHCO₃, 7.5 eq | H₂O/2-Me-THF/THF, 450mL/100mL/200mL | 89.2 | / | |
| 12 | 30 g,1 eq | 2.5 eq | | NaHCO₃, 7.5 eq | H₂O/2-Me-THF/THF, 450mL/300mL/150mL | 88.0 | 59 | 92 |
| 13 | 10 g,1 eq | 2.5 eq | | NaHCO₃, 7.5 eq | H₂O/2-Me-THF/Dioxane, 150mL/75mL/75mL | 93.5 | 56 | 98.1 |
| 14 | 30 g,1 eq | 2 eq | | NaHCO₃ 6 eq | H₂O/2-Me-THF/THF, 360mL/180mL/180mL | 90.4 | 66.4 | 96.5 |
| Comparative Example 1 | 10 g,1 eq | 2 eq | Tetrabutylammonium hydrogen sulfate, 0.1 eq | NaHCO₃, 2.5 eq | H₂O/DCM, 150mL/150mL | 30.7 | 20 | 98 |

### Example 1: Preparation of 5-nitro-8-chloromethoxyquinoline (7)

Compound 5 (1 g, 5.28 mmol, 1 eq) was placed in a 1 L round-bottomed flask, and sodium bicarbonate (2.64 g, 31.7 mmol, 6 eq) was added. Then 20 mL of water was added, and the mixture was stirred at room temperature. Then an aqueous solution of tetrabutylammonium hydroxide (0.34 g, 0.528 mmol, 0.1 eq, 40 w.t. % in water) and 20 mL of THF were added, and the mixture was stirred at room temperature for 30 minutes. Compound 6 (1.74 g, 10.56 mmol, 2.0 eq) was slowly added dropwise to the reaction flask, and the temperature was maintained at 25-30°C during the dropwise addition. The dropwise addition lasted for about 15 minutes, and the mixture was kept at 25-30°C for reaction for 1.5 hours. A large amount of compound 7 was precipitated. After that, the mixed system obtained by the reaction was analyzed by LCMS, and the result showed that the conversion rate of compound 5 was 82.7%.

Calculated MS: 238.1; measured MS: 239.1 [M+H]⁺.

### Example 2: Preparation of 5-nitro-8-chloromethoxyquinoline (7)

Compound 5 (1 g, 5.28 mmol, 1 eq) was placed in a 1 L round-bottomed flask, and sodium bicarbonate (2.64 g, 31.7 mmol, 6 eq) was added. Then 20 mL of water was added, and the mixture was stirred at room temperature. Then an aqueous solution of tetrabutylammonium hydroxide (0.34 g, 0.528 mmol, 0.1 eq, 40 w.t. % in water) and 20 mL of 2-Me-THF were added, and the mixture was stirred at room temperature for 30 minutes. Compound 6 (1.74 g, 10.56 mmol, 2.0 eq) was slowly added dropwise to the reaction flask, and the temperature was maintained at 25-30°C during the dropwise addition. The dropwise addition lasted for about 15 minutes, and the mixture was kept at 25-30°C for reaction for 1.5 hours. A large amount of compound 7 was precipitated. After that, the mixed system obtained by the reaction was analyzed by LCMS, and the result showed that the conversion rate of compound 5 was 75.2%.

Calculated MS: 238.1; measured MS: 239.1 [M+H]⁺.

### Example 3: Preparation of 5-nitro-8-chloromethoxyquinoline (7)

Compound 5 (1 g, 5.28 mmol, 1 eq) was placed in a 1 L round-bottomed flask, and sodium bicarbonate (2.64 g, 31.7 mmol, 6 eq) was added. Then 20 mL of water was added, and the mixture was stirred at room temperature. Then an aqueous solution of tetrabutylammonium hydroxide (0.34 g, 0.528 mmol, 0.1 eq, 40 w.t. % in water) and 20 mL of ethyl acetate were added, and the mixture was stirred at room temperature for 30 minutes. Compound 6 (1.74 g, 10.56 mmol, 2.0 eq) was slowly added dropwise to the reaction flask, and the temperature was maintained at 25-30°C during the dropwise addition. The dropwise addition lasted for about 15 minutes, and the mixture was kept at 25-30°C for reaction for 1.5 hours. A large amount of compound 7 was precipitated. After that, the mixed system obtained by the reaction was analyzed by LCMS, and the result showed that the conversion rate of compound 5 was 73.8%.

Calculated MS: 238.1; measured MS: 239.1 [M+H]⁺.

### Example 4: Preparation of 5-nitro-8-chloromethoxyquinoline (7)

Compound 5 (5 g, 26.3 mmol, 1 eq) was placed in a 1 L round-bottomed flask, and sodium bicarbonate (15.5 g, 197.3 mmol, 7.5 eq) was added. Then 100 mL of water was added, and the mixture was stirred at room temperature. Then an aqueous solution of tetrabutylammonium hydroxide (1.7 g, 2.63 mmol, 0.1 eq, 40 w.t. % in water) and 80 mL of THF were added, and the mixture was stirred at room temperature for 30 minutes. Compound 6 (10.9 g, 65.8 mmol, 2.5 eq) was dissolved in 20 mL of THF and slowly added dropwise to the reaction flask, and the temperature was maintained at 25-30°C during the dropwise addition. The dropwise addition lasted for about 15 minutes, and the mixture was kept at 25-30°C for reaction for 1.5 hours. A large amount of compound 7 was precipitated. After that, the mixed system obtained by the reaction was analyzed by LCMS, and the result showed that the conversion rate of compound 5 was 98.1%.

Calculated MS: 238.1; measured MS: 239.1 [M+H]⁺.

### Example 5: Preparation of 5-nitro-8-chloromethoxyquinoline (7)

Compound 5 (5 g, 26.3 mmol, 1 eq) was placed in a 1 L round-bottomed flask, and potassium bicarbonate (19.8 g, 197.3 mmol, 7.5 eq) was added. Then 100 mL of water was added, and the mixture was stirred at room temperature. Then an aqueous solution of tetrabutylammonium hydroxide (1.7 g, 2.63 mmol, 0.1 eq, 40 w.t. % in water) and 80 mL of 2-Me-THF were added, and the mixture was stirred at room temperature for 30 minutes. Compound 6 (10.9 g, 65.8 mmol, 2.5 eq) was dissolved in 20 mL of 2-Me-THF and slowly added dropwise to the reaction flask, and the temperature was maintained at 25-30°C during the dropwise addition. The dropwise addition lasted for about 15 minutes, and the mixture was kept at 25-30°C for reaction for 1.5 hours. A large amount of compound 7 was precipitated. After that, the mixed system obtained by the reaction was analyzed by LCMS, and the result showed that the conversion rate of compound 5 was 80%.

Calculated MS: 238.1; measured MS: 239.1 [M+H]⁺.

### Example 6: Preparation of 5-nitro-8-chloromethoxyquinoline (7)

Compound 5 (10 g, 52.6 mmol, 1 eq) was placed in a 3 L round-bottomed flask, and sodium bicarbonate (35.7 g, 426.2 mmol, 8.1 eq) was added. Then 200 mL of water was added, and the mixture was stirred at room temperature. Then an aqueous solution of tetrabutylammonium hydroxide (3.4 g, 5.26 mmol, 0.1 eq, 40 w.t. % in water) and 150 mL of THF were added, and the mixture was stirred at room temperature for 30 minutes. Compound 6 (23.4 g, 142.1 mmol, 2.7 eq) was dissolved in 50 mL of THF and slowly added dropwise to the reaction flask, and the temperature was maintained at 25-30°C during the dropwise addition. The dropwise addition lasted for about 15 minutes, and the mixture was kept at 25-30°C for reaction for 1.5 hours. A large amount of compound 7 was precipitated. After that, the mixed system obtained by the reaction was analyzed by LCMS, and the result showed that the conversion rate of compound 5 was 77.9%.

Calculated MS: 238.1; measured MS: 239.1 [M+H]⁺.

### Example 7: Preparation of 5-nitro-8-chloromethoxyquinoline (7)

Compound 5 (10 g, 52.6 mmol, 1 eq) was placed in a 3 L round-bottomed flask, and sodium bicarbonate (33.1 g, 394.6 mmol, 7.5 eq) was added. Then 200 mL of water was added, and the mixture was stirred at room temperature. Then an aqueous solution of tetrabutylammonium hydroxide (3.4 g, 5.26 mmol, 0.1 eq, 40 w.t. % in water) and 150 mL of THF were added, and the mixture was stirred at room temperature for 30 minutes. Compound 6 (21.7 g, 131.6 mmol, 2.5 eq) was dissolved in 50 mL of THF and slowly added dropwise to the reaction flask, and the temperature was maintained at 25-30°C during the dropwise addition. The dropwise addition lasted for about 15 minutes, and the mixture was kept at 25-30°C for reaction for 1.5 hours. A large amount of compound 7 was precipitated. After that, the mixed system obtained by the reaction was analyzed by LCMS, and the result showed that the conversion rate of compound 5 was 95.8%.

Calculated MS: 238.1; measured MS: 239.1 [M+H]⁺.

### Example 8: Preparation of 5-nitro-8-chloromethoxyquinoline (7)

Compound 5 (10 g, 52.6 mmol, 1 eq) was placed in a 3 L round-bottomed flask, and sodium bicarbonate (33.1 g, 394.6 mmol, 7.5 eq) was added. Then 200 mL of water was added, and the mixture was stirred at room temperature. Then an aqueous solution of tetrabutylammonium hydroxide (3.4 g, 5.26 mmol, 0.1 eq, 40 w.t. % in water), 100 mL of 2-Me-THF and 50 mL of THF were added, and the mixture was stirred at room temperature for 30 minutes. Compound 6 (21.7 g, 131.6 mmol, 2.5 eq) was dissolved in 50 mL of THF and slowly added dropwise to the reaction flask, and the temperature was maintained at 25-30°C during the dropwise addition. The dropwise addition lasted for about 15 minutes, and the mixture was kept at 25-30°C for reaction for 1.5 hours. A large amount of compound 7 was precipitated. After that, the mixed system obtained by the reaction was analyzed by LCMS, and the result showed that the conversion rate of compound 5 was 98.7%.

Calculated MS: 238.1; measured MS: 239.1 [M+H]⁺.

### Example 9: Preparation of 5-nitro-8-chloromethoxyquinoline (7)

Compound 5 (10 g, 52.6 mmol, 1 eq) was placed in a 3 L round-bottomed flask, and sodium bicarbonate (33.1 g, 394.6 mmol, 7.5 eq) was added. Then 150 mL of water was added, and the mixture was stirred at room temperature. Then an aqueous solution of tetrabutylammonium hydroxide (3.4 g, 5.26 mmol, 0.1 eq, 40 w.t. % in water), 75 mL of 2-Me-THF and 50 mL of THF were added, and the mixture was stirred at room temperature for 30 minutes. Compound 6 (21.7 g, 131.6 mmol, 2.5 eq) was dissolved in 25 mL of THF and slowly added dropwise to the reaction flask, and the temperature was maintained at 25-30°C during the dropwise addition. The dropwise addition lasted for about 15 minutes, and the mixture was kept at 25-30°C for reaction for 1.5 hours. A large amount of compound 7 was precipitated. After that, the mixed system obtained by the reaction was analyzed by LCMS, and the result showed that the conversion rate of compound 5 was 97.1%.

Calculated MS: 238.1; measured MS: 239.1 [M+H]⁺.

### Example 10: Preparation of 5-nitro-8-chloromethoxyquinoline (7)

Compound 5 (10 g, 52.6 mmol, 1 eq) was placed in a 3 L round-bottomed flask, and sodium bicarbonate (33.1 g, 394.6 mmol, 7.5 eq) was added. Then 100 mL of water was added, and the mixture was stirred at room temperature. Then an aqueous solution of tetrabutylammonium hydroxide (3.4 g, 5.26 mmol, 0.1 eq, 40 w.t. % in water), 50 mL of 2-Me-THF and 35 mL of THF were added, and the mixture was stirred at room temperature for 30 minutes. Compound 6 (21.7 g, 131.6 mmol, 2.5 eq) was dissolved in 15 mL of THF and slowly added dropwise to the reaction flask, and the temperature was maintained at 25-30°C during the dropwise addition. The dropwise addition lasted for about 15 minutes, and the mixture was kept at 25-30°C for reaction for 1.5 hours. A large amount of compound 7 was precipitated. After that, the mixed system obtained by the reaction was analyzed by LCMS, and the result showed that the conversion rate of compound 5 was 85.8%.

Calculated MS: 238.1; measured MS: 239.1 [M+H]⁺.

### Example 11: Preparation of 5-nitro-8-chloromethoxyquinoline (7)

Compound 5 (30 g, 157.8 mmol, 1 eq) was placed in a 3 L round-bottomed flask, and sodium bicarbonate (99.4 g, 1183.2 mmol, 7.5 eq) was added. Then 450 mL of water was added, and the mixture was stirred at room temperature. Then an aqueous solution of tetrabutylammonium hydroxide (10.24 g, 15.8 mmol, 0.1 eq, 40 w.t. % in water), 100 mL of 2-Me-THF and 150 mL of THF were added, and the mixture was stirred at room temperature for 30 minutes. Compound 6 (65.1 g, 394.4 mmol, 2.5 eq) was dissolved in 50 mL of THF and slowly added dropwise to the reaction flask, and the temperature was maintained at 25-30°C during the dropwise addition. The dropwise addition lasted for about 15 minutes, and the mixture was kept at 25-30°C for reaction for 1.5 hours. A large amount of compound 7 was precipitated. After that, the mixed system obtained by the reaction was analyzed by LCMS, and the result showed that the conversion rate of compound 5 was 89.2%.

Calculated MS: 238.1; measured MS: 239.1 [M+H]⁺.

### Example 12: Preparation of ((5-nitroquinolin-8-yl)oxy)methylisobutyryl-L-prolinate (8)

### 1. Preparation of 5-nitro-8-chloromethoxyquinoline (7)

Compound 5 (30 g, 157.8 mmol, 1 eq) was placed in a 3 L round-bottomed flask, and sodium bicarbonate (99.4 g, 1183.2 mmol, 7.5 eq) was added. Then 450 mL of water was added, and the mixture was stirred at room temperature. Then an aqueous solution of tetrabutylammonium hydroxide (10.24 g, 15.8 mmol, 0.1 eq, 40 w.t. % in water), 300 mL of 2-Me-THF and 100 mL of THF were added, and the mixture was stirred at room temperature for 30 minutes. Compound 6 (65.1 g, 394.4 mmol, 2.5 eq) was dissolved in 50 mL of THF and slowly added dropwise to the reaction flask, and the temperature was maintained at 25-30°C during the dropwise addition. The dropwise addition lasted for about 15 minutes, and the mixture was kept at 25-30°C for reaction for 1.5 hours. A large amount of compound 7 was precipitated. After that, the mixed system obtained by the reaction was analyzed by LCMS, and the result showed that the conversion rate of compound 5 was 88.0%. After that, the mixed system was filtered, the filter cake was rinsed with 50 mL of water and dried to obtain 22 g of a product as a yellow solid with a yield of 59% and a purity of 92%.

¹H-NMR (400 MHz, CDCl₃) δ: 9.18 (d, J = 8.8 Hz, 1H), 9.06 (m, 1H), 8.51 (dd, J=8.8 Hz, 1.2Hz, 1H), 7.76 (m, 1H),7.45 (d, J=1.2 Hz, 1H), 6.25 (s, 2H).

Calculated MS: 238.1; measured MS: 239.1 [M+H]⁺.

### 2. Preparation of methyl isobutyryl-L-prolinate (3)

Methyl L-prolinate hydrochloride (1) (30 g, 181.1 mmol) was placed in a 1 L three-necked flask, 300 mL of dichloromethane was added, and the mixture was cooled in an ice bath. Triethylamine (37.6 g, 371.3 mmol) was slowly added dropwise with stirring under a nitrogen atmosphere, and the mixture was stirred for 20 minutes. Then isobutyryl chloride (2) (20.3 g, 190.2 mmol) was slowly added dropwise at 0-10°C, and the mixture was stirred for 1 hour. Then the temperature was raised to 20°C for reaction for 2 hours until the reaction was stopped. After that, 30 mL of water was added, the mixture was left to stand, and liquid-liquid separation was performed to obtain an organic phase. The organic phase was concentrated under reduced pressure. The crude product obtained by concentration was dissolved in 150 mL of ethyl acetate, and then washed with 30 mL of water and 30 mL of saturated brine successively. The obtained organic phase was dried over anhydrous sodium sulfate and filtered. The filtrate was concentrated under reduced pressure to obtain 34.8 g of methyl isobutyryl-L-prolinate (3) as a colorless oil with a yield of 96.4% and a purity of 96.5%.

Calculated MS: 199.2; measured MS: 200.2 [M+H]⁺.

### 3. Preparation of isobutyryl-L-proline (4)

Methyl isobutyryl-L-prolinate (3) (34.8 g, 124.5 mmol) was dissolved in 210 mL of 2-Me-THF, 140 mL of water was added, and then LiOH·H₂O (10.4 g, 249.0 mmol) was added. The reaction solution was stirred at 20°C for 2 hours. After the reaction was stopped, the reaction solution was left to stand, and liquid-liquid separation was performed to obtain an aqueous phase. The pH of the aqueous phase was adjusted to 4-5 with aqueous HCl (35 mL, 6 N), and the aqueous phase was extracted with dichloromethane (70 mL×2). The obtained organic phase was dried over anhydrous sodium sulfate and filtered. The filtrate was concentrated under reduced pressure to obtain 29.5 g of isobutyryl-L-proline (4) as a white solid with a yield of 91.3% and a purity of 99%.

¹H-NMR (400 MHz, DMSO-d6): δ 12.47 (s,1H), 4.20-4.24 (m, 1H), 3.58-3.55 (m, 2H), 2.68-2.65 (m, 1H), 2.19-2.14 (m, 1H), 1.92-1.85 (m, 2H), 1.83-1.78 (m, 1H), 0.95 (d, J=3.6 Hz, 3H), 0.89 (d, J=3.6 Hz, 3H).

Calculated MS: 185.2; measured MS: 186.2 [M+H]⁺.

### 4. Preparation of ((5-nitroquinolin-8-yl)oxy)methylisobutyryl-L-prolinate (8)

Isobutyryl-L-proline (4) (40 g, 215.6 mmol, 1.2 eq) was dissolved in 400 mL of dry DMF, and potassium carbonate (25 g, 179.6 mmol, 1.0 eq) was added with stirring at room temperature. After stirring at room temperature for 25 minutes, 5-nitro-8-chloromethoxyquinoline (7) (42.8 g, 179.6 mmol, 1.0 eq) was added, and the mixture was kept at room temperature for reaction for 1.5 hours. After the reaction was stopped, 2 L of water was added to dilute the reaction solution, and then liquid-liquid separation was performed to obtain an organic phase. The organic phase was extracted with ethyl acetate (1 L×2), washed with 1 L of saturated brine, dried over anhydrous sodium sulfate and filtered. The filtrate was concentrated under reduced pressure to obtain 65 g of a crude product. The crude product was dissolved in 130 mL of ethyl acetate at room temperature, and was recrystallized by adding 390 mL of petroleum ether. The crystals were filtered to obtain 62 g of ((5-nitroquinolin-8-yl)oxy)methylisobutyryl-L-prolinate (8) with a yield of 99.1% and a purity of 89.2%.

¹H-NMR (400 MHz, DMSO-d6) δ: 9.05 (d, J = 4.0 Hz,1H), 9.00 (d, J = 8.8 Hz, 1H), 8.56 (d, J=8.8 Hz, 1H), 7.89-7.86 (dd, J = 4.0 Hz,8.8Hz, 1H), 7.55 (d, J=8.8 Hz, 1H), 6.24-6.11 (m, 2H), 4.36-4.33 (m, 1H), 3.58-3.55 (m, 2H), 2.68-2.65(m, 1H), 2.19-2.14(m, 1H), 1.92-1.85 (m, 2H), 1.83-1.78 (m, 1H), 0.95 (d, J=6.8 Hz, 3H), 0.89 (d, J=6.8 Hz, 3H).

Calculated MS: 387.3; measured MS: 388.3 [M+H]⁺.

### Example 13: Preparation of 5-nitro-8-chloromethoxyquinoline (7)

Compound 5 (10 g, 52.6 mmol, 1 eq) was placed in a 3 L round-bottomed flask, and sodium bicarbonate (33.1 g, 394.6 mmol, 7.5 eq) was added. Then 150 mL of water was added, and the mixture was stirred at room temperature. Then an aqueous solution of tetrabutylammonium hydroxide (3.4 g, 5.26 mmol, 0.1 eq, 40 w.t. % in water), 75 mL of 2-Me-THF and 50 mL of THF were added, and the mixture was stirred at room temperature for 30 minutes. Compound 6 (21.7 g, 131.6 mmol, 2.5 eq) was dissolved in 25 mL of THF and slowly added dropwise to the reaction flask, and the temperature was maintained at 25-30°C during the dropwise addition. The dropwise addition lasted for about 15 minutes, and the mixture was kept at 25-30°C for reaction for 1.5 hours. A large amount of compound 7 was precipitated. After that, the mixed system obtained by the reaction was analyzed by LCMS, and the result showed that the conversion rate of compound 5 was 93.5%. After that, the mixed system was filtered, the filter cake was rinsed with 50 mL of water and dried to obtain 7 g of a product as a yellow solid with a yield of 56% and a purity of 98.1%.

1H-NMR (400 MHz, CDCl3) δ: 9.18 (d, J = 8.8 Hz, 1H), 9.06 (m,1H), 8.51 (dd, J=8.8 Hz, 1.2Hz,1H), 7.76 (m, 1H),7.45 (d, J=1.2 Hz, 1H), 6.25 (s, 2H).

Calculated MS: 238.1; measured MS: 239.1 [M+H]⁺.

### Example 14: Preparation of 5-nitro-8-chloromethoxyquinoline (7)

Compound 5 (30 g, 157.8 mmol, 1 eq) was placed in a 3 L round-bottomed flask, and sodium bicarbonate (79.5 g, 947 mmol, 6 eq) was added. Then 360 mL of water was added, and the mixture was stirred at room temperature. Then an aqueous solution of tetrabutylammonium hydroxide (10.24 g, 15.8 mmol, 0.1 eq, 40 w.t. % in water), 180 mL of 2-Me-THF and 140 mL of THF were added, and the mixture was stirred at room temperature for 30 minutes. Compound 6 (52.1 g, 315.5 mmol, 2 eq) was dissolved in 40 mL of THF and slowly added dropwise to the reaction flask, and the temperature was maintained at 25-30°C during the dropwise addition. The dropwise addition lasted for about 15 minutes, and the mixture was kept at 25-30°C for reaction for 1.5 hours. A large amount of compound 7 was precipitated. After that, the mixed system obtained by the reaction was analyzed by LCMS, and the result showed that the conversion rate of compound 5 was 90.4%. After that, the mixed system was filtered, the filter cake was rinsed with 50 mL of water and dried to obtain 25 g of a product as a yellow solid with a yield of 66.4% and a purity of 96.5%.

¹H-NMR (400 MHz, CDCl₃) δ: 9.18 (d, J = 8.8 Hz, 1H), 9.06 (m,1H), 8.51 (dd, J=8.8 Hz, 1.2Hz,1H), 7.76 (m, 1H),7.45 (d, J=1.2 Hz, 1H), 6.25 (s, 2H).

Calculated MS: 238.1; measured MS: 239.1 [M+H]⁺.

### Comparative Example 1: Preparation of ((5-nitroquinolin-8-yl)oxy)methylisobutyryl-L-prolinate (8)

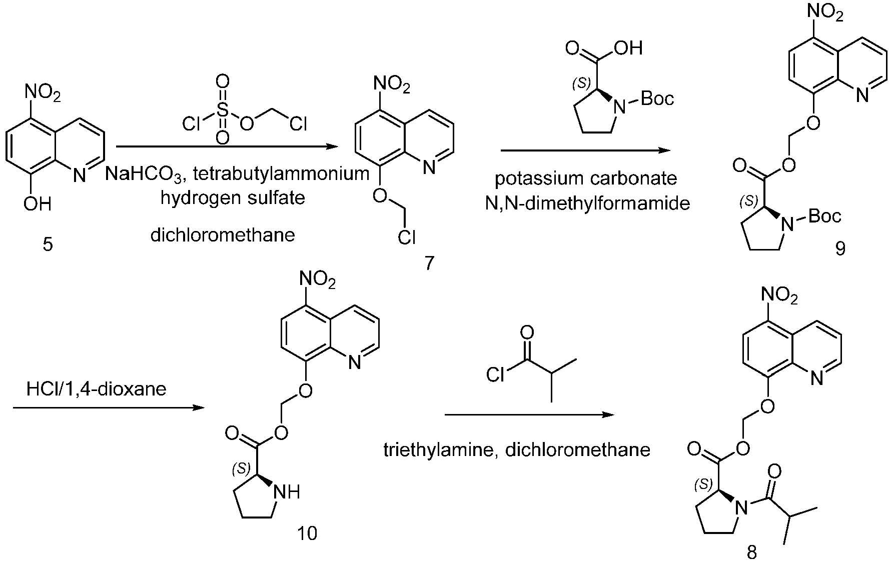

### Step 1: Preparation of 5-nitro-8-chloromethoxyquinoline (7)

An aqueous solution of sodium bicarbonate (150 mL, 0.88 mol/L) and tetrabutylammonium hydrogen sulfate (1.78 g, 5.24 mmol) were added to a solution of nitroxoline (5) (10.00 g, 52.59 mmol) in dichloromethane (150 mL) at room temperature, and the mixture was stirred for 20 minutes. Chloromethyl chlorosulfonate (17.44 g, 105.7 mmol) was added dropwise to the reaction system, which was stirred at room temperature for 16 hours. After that, the mixed system obtained by the reaction was analyzed by LCMS, and the result showed that the conversion rate of compound 5 was 30.7%. After that, the reaction solution was filtered, and liquid-liquid separation was performed to obtain an organic phase. The organic phase was washed successively with a saturated solution of potassium carbonate (20 mL) and saturated brine (20 mL), dried over anhydrous sodium sulfate and filtered. The filtrate was concentrated under reduced pressure. Then the obtained solid was purified by silica gel column chromatography (eluent: dichloromethane: methanol=20:1, the ratio was a volume ratio) to obtain 2.5 g of 5-nitro-8-chloromethoxyquinoline (7) with a yield of 20% and a purity of 98%.

¹H-NMR (400 MHz, CDCl₃) δ: 9.18 (d, J = 8.8 Hz, 1H), 9.06 (m,1H), 8.51 (dd, J=8.8 Hz, 1.2Hz, 1H), 7.76 (m, 1H), 7.45 (d, J=1.2 Hz, 1H), 6.25 (s, 2H).

Calculated MS: 238.1; measured MS: 239.1 [M+H]⁺.

### Step 2: Preparation of 1-(tert-butyl) 2-(((5-nitroquinolin-8-yl)oxy)methyl) (S)-pyrrolidine-1,2-dicarboxylate (9)

5-Nitro-8-chloromethoxyquinoline (7) (1.5 g, 6.3 mmol) and Boc-L-proline (2.02 g, 9.4 mmol) were dissolved in 15 mL of DMF at room temperature, and potassium carbonate (1.73 g, 12.6 mmol) was added for reaction at room temperature for 3 hours. 70 mL of water was added to the reaction solution, and liquid-liquid separation was performed to obtain an organic phase. The organic phase was extracted with ethyl acetate (50 mL×2), washed with saturated brine, dried over anhydrous sodium sulfate and filtered. The filtrate was concentrated under reduced pressure. The residue was purified by silica gel column chromatography (eluent: PE:EA = 1:1, the ratio was a volume ratio) to obtain 2.8 g of 1-(tert-butyl) 2-(((5-nitroquinolin-8-yl)oxy)methyl) (S)-pyrrolidine-1,2-dicarboxylate (9) as a yellow oil with a yield of 106% and a purity of 97%.

Calculated MS: 417.1; measured MS: 418.2 [M+H]⁺.

### Step 3: Preparation of ((5-nitroquinolin-8-yl)oxy)methyl-L-prolinate hydrochloride (10)

1-(Tert-butyl) 2-(((5-nitroquinolin-8-yl)oxy)methyl) (S)-pyrrolidine-1,2-dicarboxylate (9) (2.8 g, 6.71 mmol) was placed in 30 mL of a solution of HCl in dioxane (4 M) at 0°C, and the mixture was stirred at room temperature for 20 minutes. The reaction solution was concentrated under reduced pressure to obtain 2.3 g of ((5-nitroquinolin-8-yl)oxy)methyl L-prolinate hydrochloride (10) as a white solid with a yield of 97% and a purity of 96%.

Calculated MS: 317.1; measured MS: 318.1 [M+H]⁺.

### Step 4: Preparation of ((5-nitroquinolin-8-yl)oxy)methylisobutyryl-L-prolinate (8)

((5-Nitroquinolin-8-yl)oxy)methyl-L-prolinate hydrochloride (10) (150 mg, 0.43 mmol) was placed in 10 mL of DCM at room temperature and cooled to 0-5°C in an ice bath. Isobutyryl chloride (0.86 mmol) was added, and then TEA (170 mg, 1.72 mmol) was slowly added. After the addition was completed, the reaction solution was stirred at room temperature for 20 minutes and concentrated under reduced pressure. The residue was purified by silica gel column chromatography (eluent: PE:EA=1:1, the ratio was a volume ratio) to obtain 41 mg of ((5-nitroquinolin-8-yl)oxy)methylisobutyryl-L-prolinate (8) with a yield of 24.6% and a purity of 99%.

¹H-NMR (400 MHz, DMSO-d6) δ: 9.05 (d, J = 4.0 Hz,1H), 9.01 (d, J = 8.8 Hz, 1H), 8.58 (d, J=8.8 Hz, 1H), 7.80-7.86 (dd, J = 4.0 Hz, 8.8Hz, 1H), 7.55 (d, J=8.8 Hz, 1H), 6.22-6.14 (m, 2H), 4.36-4.33 (m, 1H), 3.55-3.55 (m, 2H), 2.68-2.65 (m, 1H), 2.19-2.13 (m, 1H), 1.92-1.85 (m, 2H), 1.86-1.78 (m, 1H), 0.94 (d, J=6.8 Hz, 3H), 0.89 (d, J=6.8 Hz, 3H).

Calculated MS: 387.3; measured MS: 388.3 [M+H]⁺.

According to the relevant experimental data in Table 1, it can be seen that the preparation method for the aromatic ether compound of the present invention can greatly improve the conversion rate of the reaction of compound 5 and compound 6 to prepare compound 7, thereby improving the yield of this step and the overall yield of the entire reaction route (the reaction route for producing compound 8).

In addition, when the reaction of compound 5 and compound 6 to prepare compound 7 is completed, a large amount of compound 7 is precipitated in the reaction system. Thus, the preparation method for the aromatic ether compound of the present invention greatly simplifies the post-treatment and purification operations for preparing compound 7. The compound 7 with a purity of more than 92%, even more than 98% in some examples, can be obtained by simply filtering the reaction solution and washing and drying the filter cake, and the compound 7 in the filtrate can also be extracted by simply concentration and crystallization, thereby avoiding the industrially difficult purification means such as column chromatography. Therefore, the above-mentioned preparation method of the present invention is suitable for industrial scale-up production. Here, the inventors of the present invention also wish to explain that the chloromethyl ether moiety contained in compound 7 is sensitive to acid and base as well as heat, making the preparation and purification very difficult, and there are few reports in published literature. The inventors of the present invention have done a great deal of research to develop the very advantageous preparation method for compound 7 of the present invention.

## Claims

1. A preparation method for a compound of formula VII, **characterized in that**, the preparation method comprises the following step of:
c) reacting compound V with compound 6 in a solvent in the presence of a catalyst and a base to obtain the compound VII, 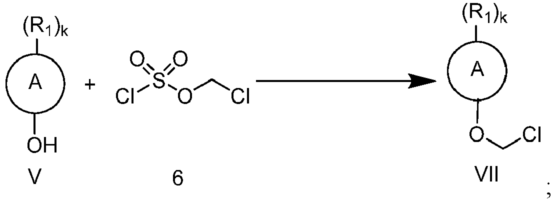
wherein, ring A is an aromatic ring or a heteroaromatic ring, preferably a 6- to 10-membered aromatic ring or a 5- to 10-membered heteroaromatic ring, more preferably a benzene ring, a naphthalene ring, a pyridine ring or a quinoline ring; the hydroxyl group is attached to a carbon on ring A;
each R₁ is independently selected from the group consisting of -R₂, 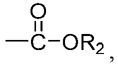 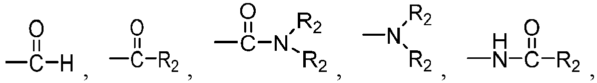 -NO₂, -NO, 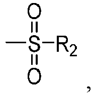 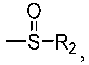 -S-R₂, -OR₂ and -X; wherein, each R₂ is independently C₁-C₂₀ alkyl, preferably C₁-C₆ alkyl, more preferably methyl, ethyl, n-propyl or isopropyl; X is halogen, preferably fluorine, chlorine, bromine or iodine;
k is from 0 to the maximum number available for substitution on ring A; preferably, k is an integer from 0 to 6; more preferably, k is an integer from 0 to 4; further preferably, k is 0, 1 or 2; most preferably, k is 1;
the solvent is a binary or more solvent system in which the solvent used is selected from the group consisting of water, tetrahydrofuran, 2-methyltetrahydrofuran, ethyl acetate and dioxane.

2. The preparation method for the compound of formula VII according to claim 1, **characterized in that**, in step c), the compound V is 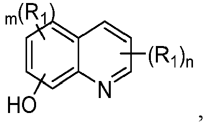 m and n are each independently 0, 1, 2 or 3; preferably 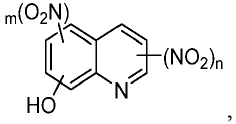 m and n are each independently 0, 1, 2 or 3; more preferably 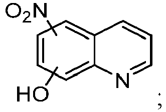 further more preferably

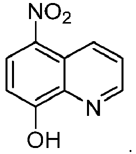

3. The preparation method for the compound of formula VII according to claim 1, **characterized in that**,
in step c), the compound V is 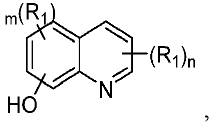 the compound VII is 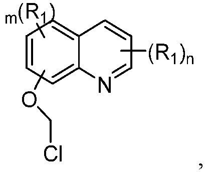 m and n are each independently 0, 1, 2 or 3;
preferably, the compound V is 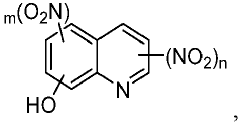 the compound VII is 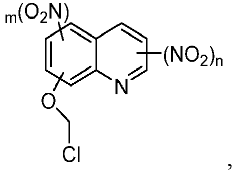 m and n are each independently 0, 1, 2 or 3;
more preferably, the compound V is 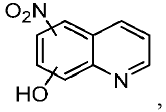 the compound VII is 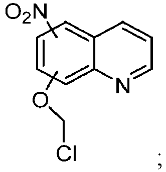
further more preferably, the compound V is 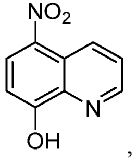 the compound VII is

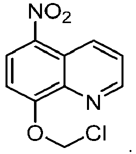

4. The preparation method for the compound of formula VII according to any one of claim 1 to 3, **characterized in that**, in step c), the solvent is a binary solvent system or ternary solvent system in which the solvent used is selected from the group consisting of water, tetrahydrofuran, 2-methyltetrahydrofuran, ethyl acetate and dioxane.

5. The preparation method for the compound of formula VII according to any one of claim 1 to 3, **characterized in that**, in step c), the solvent is any one of the binary solvent systems of water/tetrahydrofuran, water/2-methyltetrahydrofuran and water/ethyl acetate; or, any one of the ternary solvent systems of water/2-methyltetrahydrofuran/tetrahydrofuran and water/2-methyltetrahydrofuran/dioxane; the solvent is preferably a binary solvent system of water/tetrahydrofuran or a ternary solvent system of water/2-methyltetrahydrofuran/tetrahydrofuran; more preferably a ternary solvent system of water/2-methyltetrahydrofuran/tetrahydrofuran;
in the binary solvent system, the volume ratio of water to the organic solvent is preferably 10:15 to 15:0.1, more preferably 0.8:1 to 1.2:1, further more preferably 1:1;
in the ternary solvent system, the volume ratio of the three is preferably 10:15:15 to 15:5:5, more preferably 3:2:1 to 2.25:0.5:1, further more preferably 2:1:1.

6. The preparation method for the compound of formula VII according to any one of claim 1 to 5, **characterized in that**,
in step c), the catalyst is a quaternary ammonium phase transfer catalyst, preferably one or more of tetrabutylammonium hydroxide, tetrabutylammonium acetate, tetrabutylammonium hydrogen sulfate and tetrabutylammonium chloride, more preferably tetrabutylammonium hydroxide;
preferably, in step c), the base is one or both of sodium bicarbonate and potassium bicarbonate, preferably sodium bicarbonate;
preferably, in step c), the molar ratio of the compound V to the catalyst is 1:0.01 to 1:0.3, preferably 1:0.05 to 1:0.2, more preferably 1:0.1;
preferably, in step c), the molar ratio of the compound V to the base is 1:2.5 to 1:15, preferably 1:6 to 1:10, more preferably 1:6 to 1:8, further more preferably 1:7 to 1:8;
preferably, in step c), the molar ratio of the compound V to the compound 6 is 1:1 to 1:5, preferably 1:2 to 1:3, more preferably 1:2.5 to 1:2.7;
preferably, in step c), the volume/mass ratio of the solvent to the compound V is 20 mL/g to 50 mL/g, preferably 24 mL/g to 45 mL/g, more preferably 25 mL/g to 40 mL/g, further more preferably 25 mL/g to 35 mL/g, still further more preferably 30 mL/g;
preferably, in step c), the reaction temperature of the reaction is 20°C to 35°C, preferably 25°C to 30°C.

7. The preparation method for the compound of formula VII according to any one of claim 1 to 6, **characterized in that**, the preparation method further comprises the following step before step c):
b) hydrolyzing compound 3 in a solvent in the presence of a base to obtain compound 4, 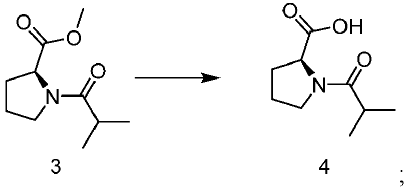
in step b), the solvent is preferably a mixed solvent of 2-methyltetrahydrofuran and water; the base is preferably lithium hydroxide;
preferably, the preparation method further comprises the following step before step b):
a) reacting compound 1 with compound 2 in a solvent in the presence of a base to obtain compound 3, 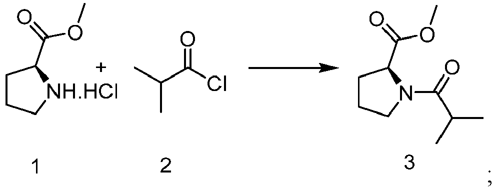 in step a), the solvent is preferably dichloromethane; the base is preferably one or both of triethylamine and diisopropylethylamine, more preferably triethylamine.

8. A preparation method for a compound of formula VIII, **characterized in that**, the preparation method comprises the following step of:
d) reacting compound 4 with compound VII in a solvent in the presence of a base to obtain the compound VIII, 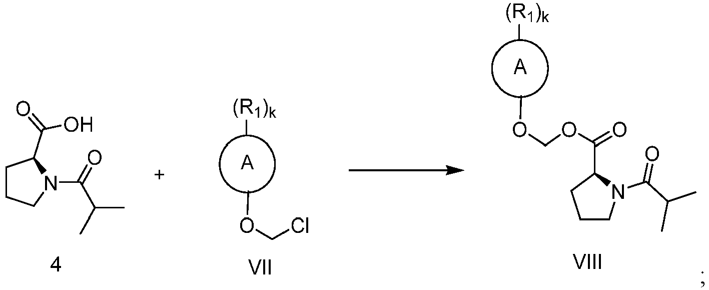
wherein, ring A is an aromatic ring or a heteroaromatic ring, preferably a 6- to 10-membered aromatic ring or a 5- to 10-membered heteroaromatic ring, more preferably a benzene ring, a naphthalene ring, a pyridine ring or a quinoline ring; -OCH₂Cl is attached to a carbon on ring A;
each R₁ is independently selected from the group consisting of -R₂, 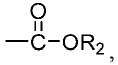 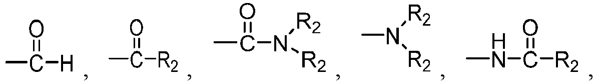 -NO₂, -NO, 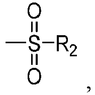 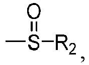 -S-R₂, -OR₂ and -X; wherein, each R₂ is independently C₁-C₂₀ alkyl, preferably C₁-C₆ alkyl, more preferably methyl, ethyl, n-propyl or isopropyl; X is halogen, preferably fluorine, chlorine, bromine or iodine;
k is from 0 to the maximum number available for substitution on ring A; preferably, k is an integer from 0 to 6; more preferably, k is an integer from 0 to 4; further preferably, k is 0, 1 or 2; most preferably, k is 1;
the compound VII is prepared according to the preparation method for the compound of formula VII according to any one of claims 1 to 7;
in step d), the solvent is preferably one or more of DMF, NMP and ACN, more preferably DMF;
in step d), the base is preferably one or more of potassium carbonate, cesium carbonate and sodium carbonate, more preferably potassium carbonate;
in step d), the molar ratio of the compound 4 to the base is preferably 1-1.5:1, more preferably 1.2:1;
in step d), the molar ratio of the compound 4 to the compound VII is preferably 1-1.5:1, more preferably 1.2:1;
in step d), the ratio of the volume of the solvent to the mass of the compound 4 is preferably 8:1 mL/g to 12:1 mL/g, more preferably 10:1 mL/g;
in step d), the reaction temperature of the reaction is preferably 20°C to 30°C.

9. The preparation method for the compound of formula VIII according to claim 8, **characterized in that**, the preparation method also comprises a step e) of separating and purifying the compound VIII after step d); the step e) preferably comprises the following steps of: dissolving the compound VIII obtained in step d) in a positive solvent, and then mixing the solution with an anti-solvent to obtain a crystal form of compound VIII;
wherein, the positive solvent is preferably ethyl acetate; the anti-solvent is preferably petroleum ether.

10. The preparation method for the compound of formula VIII according to claim 8 or 9, **characterized in that**, the compound VII is 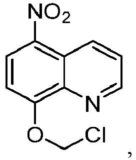 the compound VIII is
